# EUROPEAN PATENT APPLICATION

(11) **EP 4 755 419 A2**
(43) Date of publication of application: **10.06.2026**
(21) Application number: 26173756.3
(22) Date of filing: 17.02.2022
(51) Int. Cl.: A61M 5/315

(54) **IMPROVED DRIVE MECHANISMS FOR POSITIVE DISPLACEMENT PUMPS**

(30) Priority: 12.03.2021 US 202163160240 P; 04.02.2022 US 202263306765 P
(62) Divisional of application: 22708661.8
(71) Applicant: Insulet Corporation, Acton, MA 01720 (US)
(72) Inventor: KAMRAVA, Soroush, Everett, 02149 (US); CARDINALI, Steven, Tewksbury, 01876 (US); QUINN, Ian, Concord, 01742 (US)
(74) Representative: Peterreins Schley

(57) **Abstract**

A novel embodiment of a pump system, for example, of the type that would be used in a wearable drug delivery system, comprises, in a preferred embodiment, a reservoir, a plunger, disposed within the reservoir and driven by a scissor mechanism connected to a drive mechanism through a linkage, such that a force imparted by the drive mechanism in causes the scissor mechanism to expand thereby causing the plunger move within the reservoir to force a liquid drug contained within the reservoir through a fluid port to a patient.

## Description

### RELATED APPLICATIONS

This application claims the benefit of U.S. Provisional Patent Application No. 63/160,240, filed March 12, 2021, entitled "IMPROVED DRIVE MECHANISMS FOR POSITIVE DISPLACEMENT PUMPS", and U.S. Provisional Patent Application No. 63/306,765, filed February 4, 2022, entitled "PUMPING MECHANISM WITH WIRE-PULLED PLUNGER". The contents of these application are incorporated herein in their entireties.

### BACKGROUND

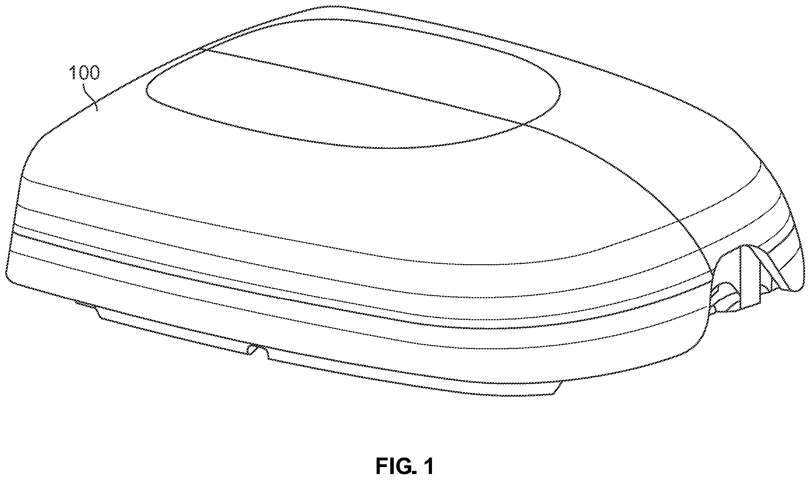

Many conventional drug delivery systems are well known, including, for example, wearable drug delivery devices of the type shown in **FIG. 1****.** The drug delivery device 100 can be designed to deliver any type of liquid drug to a user. As used herein, the term "liquid drug" is meant to include, any liquid drug, medicine, therapeutic agent, or infusate, including, for example, insulin, GLP-1, Pramlintide, or co-formulations thereof.

The drug delivery device 100 can be a single-use device (e.g., filled once and used once and then discarded) or can be a multiple-use device (e.g., filled one or more times and used after one or more fillings). In specific embodiments, the drug delivery device 100 can be, for example, an OmniPod^{®} drug delivery device manufactured by Insulet Corporation of Acton, Massachusetts. The drug delivery device 100 can be a drug delivery device such as those described in U.S. Pat. No. 7,303,549, U.S. Pat. No. 7,137,964, or U.S. Pat. No. 6,740,059, each of which is incorporated herein by reference in its entirety.

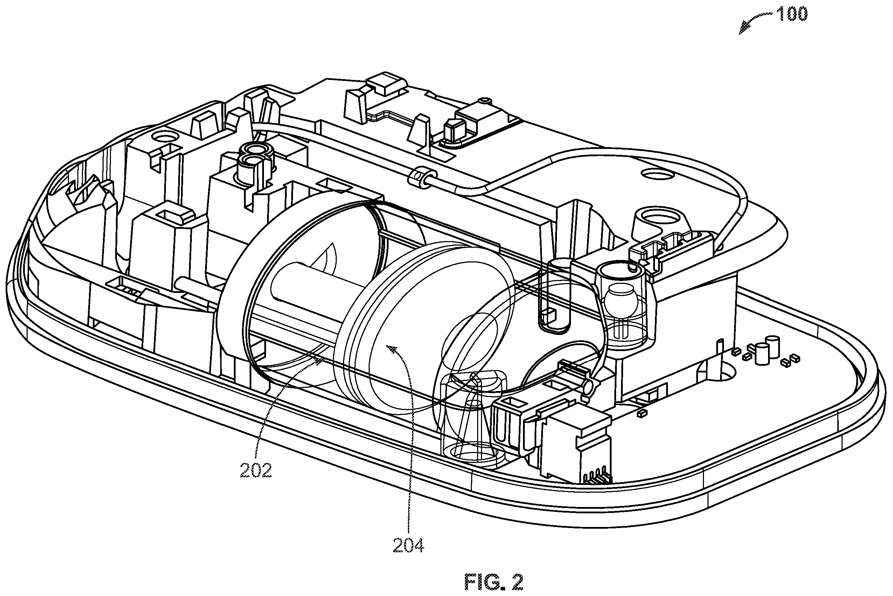

**FIG. 2** illustrates an exemplary drug delivery device 100 of the type shown in **FIG. 1** with the cover removed. Drug delivery device 100 typically includes a pump system which includes a drug container, often referred to as a reservoir 202, that stores the liquid drug. The liquid drug stored in the reservoir 202 may be delivered to the user by expelling the drug from reservoir 202 using a driven plunger 204, for example, a leadscrew driven plunger.

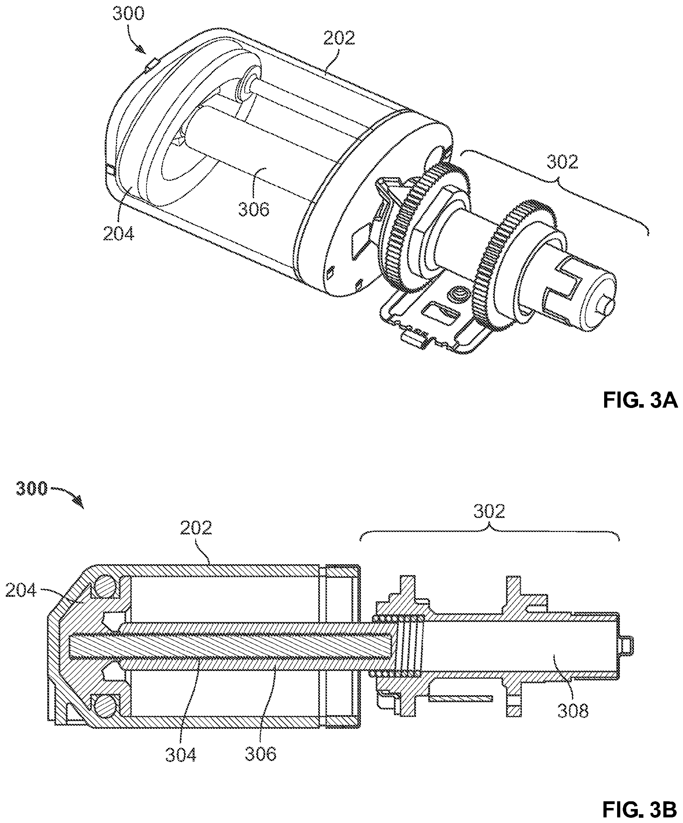

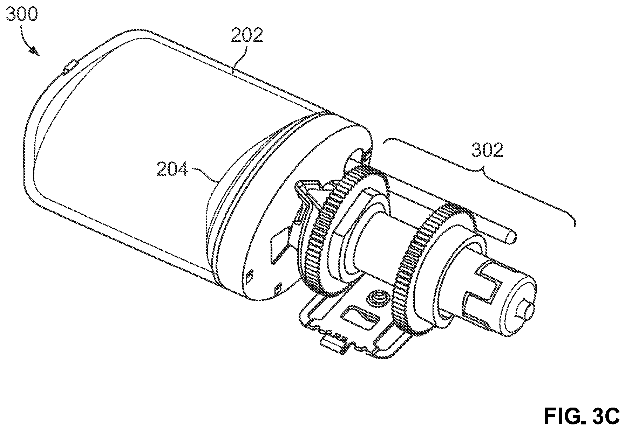

An exemplary pump system 300 is shown in perspective view in **FIG. 3A** and in cross-sectional view in **FIG. 3B****.** Pump system 300 may comprise a drive mechanism 302 capable of providing a rotational motion which will cause a tube nut 306 to rotate. Tube nut 306 is in threaded engagement with leadscrew 304 such that when tube nut 306 is rotated by drive mechanism 302, leadscrew 304 moves in a longitudinal direction within reservoir 202. Because leadscrew 304 is coupled to plunger 204, longitudinal movement of leadscrew 304 will cause plunger 204 to move longitudinally within reservoir 202. Note that **FIGS. 3A** and **3B** show reservoir 202 in an empty state, wherein plunger 204 is positioned against the distal end wall of reservoir 202. **FIG. 3C** shows reservoir 202 in a full state, wherein plunger 204 is positioned at the proximal end of reservoir 202. Note that, when the reservoir 202 is in a full or partially-full state, leadscrew 304 must occupy space 308, shown in .**FIG. 3B****.**

One limitation of this design is that the total footprint of the reservoir 202 and drive mechanism 302 is greater than the length of reservoir 202 by as much as 2 times. This is due to the fact that the leadscrew 304 needs to reach all the way into reservoir 202 when reservoir 202 is in the empty state (i.e. it must be approximately equal to the length of reservoir 202 minus space taken by plunger 204). When reservoir 202 is full, leadscrew 304 will necessarily extend behind reservoir 202 to occupy space 308 at a length up to the length of the reservoir.

In wearable, on-body devices, it is desirable to keep pump mechanism 300, as well as the overall drug delivery device 100, as small as possible to minimize the impact to the wearer. Therefore, it would be desirable to replace the prior art pump system 300 with a positive displacement pump system having a different method of driving the plunger 204 within the reservoir that does not require the large footprint of the prior art pump mechanism 300. This would reduce the size footprint of pump mechanism 300, thereby improving the size impact of the overall drug delivery system 100 while maintaining the benefits of the pumping methodology.

### BRIEF DESCRIPTION OF THE DRAWINGS

**FIG. 1** is a perspective view of the wearable drug delivery system of the type with which the present invention may be used.
**FIG. 2** is a perspective view of the wearable drug delivery system of **FIG. 1** having the cover removed to reveal the arrangement of internal parts.
**FIG. 3A** is a perspective view of a pump mechanism utilized in the drug delivery system of **FIG. 1****,** showing the positioning of various parts of the pump mechanism when the reservoir is in an empty state.
**FIG. 3B** is a cross-sectional view of the pump mechanism of **FIG. 3A** showing the positioning of the leadscrew responsible for moving the plunger within the reservoir.
**FIG. 3C** is a perspective view of the pump mechanism of **FIG. 3A** showing the positioning of various parts of the pump mechanism when the reservoir is in a full state.
**FIGS. 4A** and **4B** are side cutaway views and **FIGS. 4C** and **4D** are upright and inverted perspective cutaway views respectively of a first, preferred embodiment of the invention in which a scissor linkage is used to move the plunger through the reservoir.
**FIGS. 5A** and **5B** are upright and inverted cutaway views respectively of a variant of the embodiment of **FIGS 4A-4D****.**
**FIGS. 6A** and **6B** are side cutaway views and **FIGS. 6C** and **6D** are upright and inverted perspective views respectively of a second embodiment of the invention in which coupled dual scissor linkages are used to move the plunger through the reservoir.
**FIG. 7** is a view of another embodiment of the invention showing a different configuration for the scissor linkage.
**FIG. 8** is a side cross-sectional view of a third embodiment of the invention in which a magnetic linkage is used to move the plunger through the reservoir.
**FIG. 9** is a cross-sectional view of a fourth embodiment of the invention in which the plunger is moved through the reservoir using a flexible tether linkage wound about a take-up reel by pulling the plunger toward the distal end of the reservoir.
**FIGS. 10****(A-C)** show a fifth embodiment of the invention in which the plunger is moved through the reservoir using a flexible tether linkage wound about a take-up reel by pushing the plunger toward the distal end of the reservoir.

### DETAILED DESCRIPTION

This disclosure presents various systems, components and methods for moving a liquid, typically a liquid drug, such as insulin or GLP-1, from a liquid reservoir in a wearable drug delivery device to a patient interface, typically a needle or cannula. Each of the systems, components and methods disclosed herein provides one or more advantages over conventional, prior art systems, components and methods.

In various embodiments of the invention, the reservoir and pump are integrated into a single component (referred to herein as the "pump mechanism") and the drive mechanism of the prior art pump mechanism is replaced by a new drive mechanism which reduces the overall size footprint of the pump mechanism.

All embodiments of the pump mechanisms described herein comprise various components including a reservoir, a plunger configured to translate longitudinally through the interior of the reservoir, a drive mechanism and a linkage between the drive mechanism and the plunger. In all described embodiments, the reservoir may comprise a tube-like structure having a proximal, open and a distal, closed-end, wherein the closed-end may be configured with a fluid path such as to allow the liquid drug disposed between the plunger and the closed end of the reservoir to be forced through the fluid path to a patient interface. The reservoir may, in preferred embodiments of the invention, be composed of a polyethylene or an injection-molded plastic, but, in other embodiments, may be composed of any material impermeable to the liquid drug disposed therein. The plunger may be composed of any material and may be configured with one or more O-rings along a circumferential surface such as to create a seal between the plunger and the reservoir when the plunger is disposed within the reservoir. The cross-sectional area of the reservoir and plunger may be of any convenient shape; however, in preferred embodiments, the cross-sectional shape of both the reservoir and the plunger is oval or circular.

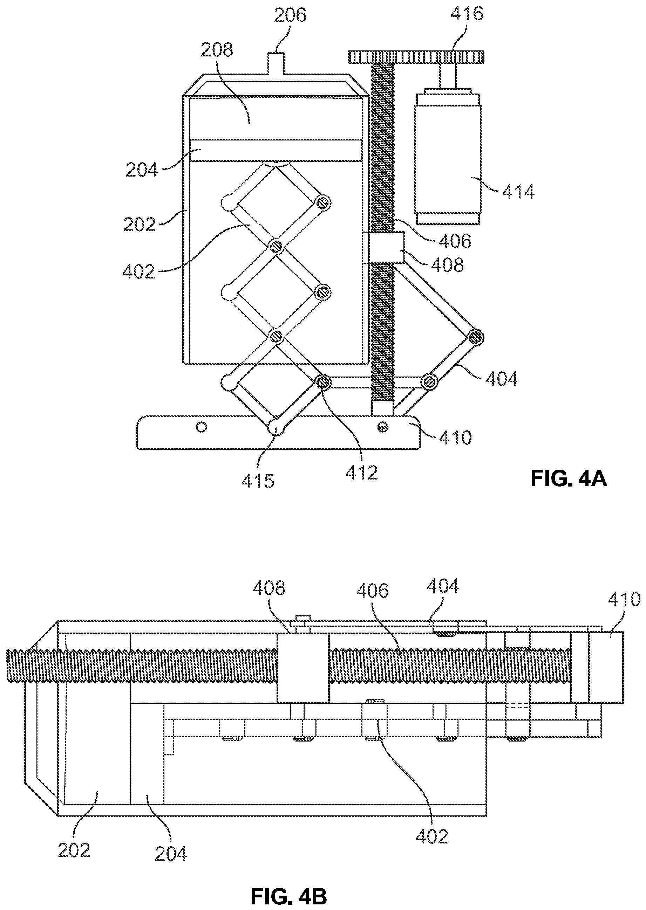

A first, preferred embodiment of the invention is shown in various views in **FIGS. 4A-4D** and illustrates a pump mechanism provided as part of a drug delivery device for delivery of a liquid drug to a user.

Reservoir 202 stores the liquid drug prior to delivery to the user. In some embodiments, the drug delivery device may come with a prefilled reservoir containing the liquid drug. In other embodiments, the reservoir 202 may be filled or refillable by the user. Reservoir 202 may be fitted with a plunger 204 which is longitudinally translatable through the interior length of reservoir 202. The liquid drug is stored in the area 208 of reservoir 202 between plunger 204 and the distal end of reservoir 202. Longitudinal translation of plunger 204 toward the distal end of reservoir 208 will force the liquid drug from area 208 via fluid path 206 to a patient interface, typically a needle or cannula (not shown). Fluid path 206 may be provided with a one-way valve that prevents fluids from entering area 208 of reservoir 202 (not shown). Fluid path 206 may be located at any point such as to be in fluid communication with space 208 within reservoir 202; however, in preferred embodiments, fluid path 206 is located as close to the distal end of reservoir 202 as possible to avoid wasting any liquid drug that would otherwise get trapped at the far distal end of the reservoir. Preferably, reservoir 202 is rigidly attached to the body of the drug delivery device.

The longitudinal translation of the plunger 204 within reservoir 202, in the preferred embodiments, is accomplished via a scissor mechanism 402. Scissor mechanism 402 is attached, at one end, to plunger 204 and at the other end, to support structure 410. Support structure 410 may be rigidly attached to the body of the drug delivery device 100 or may be part of and integral with the body of the drug delivery device 100. As such, references to "the body of the drug delivery device" herein are also meant to refer to embodiments in which support structure 410 is separate from and attached to the body of the drug delivery device. As such, reference number 410 may hereinafter refer to a support structure attached to the body of the device or to the body of the device directly.

Reservoir 202 should be rigidly coupled to the body 410 of the device such as to prevent movement between reservoir 202 and the body 410 of the device. Additionally, one end of scissor mechanism 402 should be rigidly attached to the body 410 of the device such as to prevent relative movement between the end of the scissor mechanism 402 attached to the body 410 of the device and the reservoir 202. As such, the expansion of scissor mechanism 402 will drive plunger 204 toward the distal end of reservoir 202, thereby expelling a quantity of the liquid drug contained within space 208 through fluid path 206.

The mechanical motion needed to expand or contract scissor mechanism 402 is provided by a drive mechanism. In the preferred embodiment, the drive mechanism may comprise leadscrew 406 and a drive nut 408. In other embodiments, the drive mechanism may be any mechanism capable of imparting a force to scissor mechanism 402 necessary to expand scissor mechanism 402 and to overcome any resistance from the liquid drug located in area 208 of reservoir 202. In the case wherein the drive mechanism is as shown in **FIGS. 4A-4D****,** drive nut 408 is in threaded engagement with leadscrew 406 such that rotational motion of leadscrew 406 translates to a linear motion of drive nut 408. Leadscrew 406 may be rotationally driven via any known means, including, for example, by a motor 414 coupled directly or via gearing 416 to leadscrew 406.

Drive nut 408 may be coupled to scissor mechanism 402 via a linkage 404 which is coupled to drive nut 408 and to scissor mechanism 402 at connection point 412. In addition, linkage 404 may have one or more attachments to the body 410 of the device.

As shown in **FIGS. 4A-4D****,** in some embodiments of the invention, linkage 404 may be a four-segment linkage driven by drive nut 408 which translates the linear motion of drive nut 408 into a circular or elliptical motion at connection point 412. Other linkage designs may also be used and are considered to be within the scope of the invention.

As such, the linear motion of drive nut 408 imparts a mechanical force through linkage 404 such as to cause the expansion or contraction of scissor mechanism 402. Linear motion of the drive nut 408 in one direction may cause the expansion of scissor mechanism 402, while linear motion of the drive nut 408 in the opposite direction may cause the contraction of scissor mechanism 402. In the arrangement shown in **FIGS. 4A-4D****,** linear motion of drive nut 408 toward the body 410 of the device will cause the contraction of scissor mechanism 402, while linear motion of drive nut 408 in the opposite direction, away from the body 410 of the device, will cause expansion of scissor mechanism 402.

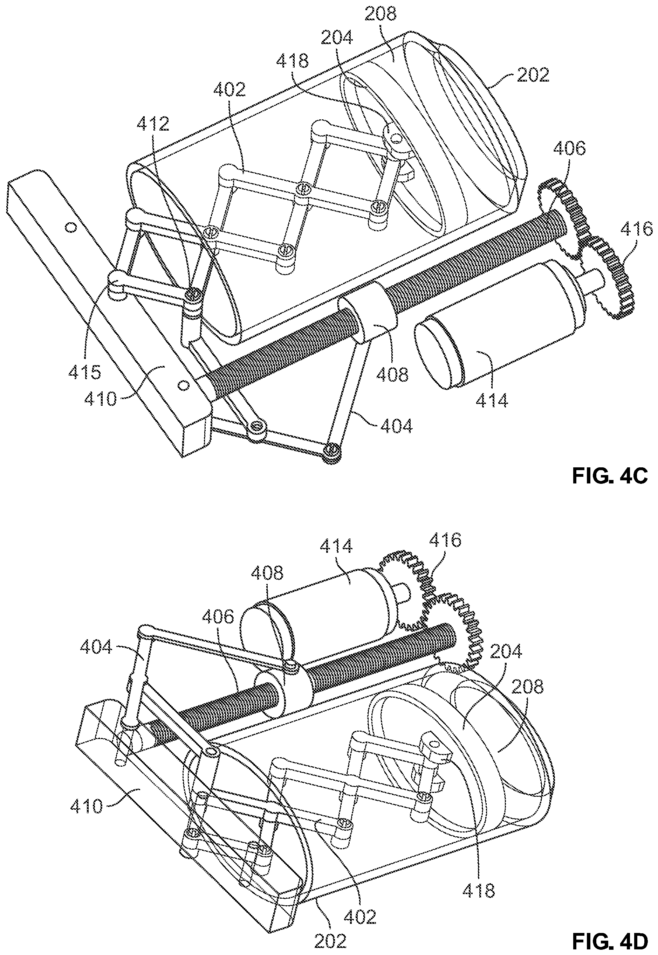

As can be seen in **FIG. 4D****,** scissor mechanism 402 is attached to plunger 204 at connection point 418. In preferred embodiments of the invention, connection point 418 is rotationally attached to plunger 204 to allow for movement of the upper segments of scissor mechanism 402 as it expands or contracts. Additionally, as shown in **FIG. 4C****,** the opposite end of scissor mechanism 402 is rotationally attached to the body 410 of the device at connection point 415.

Leadscrew 406, drive nut 408, scissor mechanism 402 and linkage 404 may, in some preferred embodiments, be composed of an injection-molded plastic or a metal, for example, stainless steel. However, these components may also be composed of any materials capable of withstanding the required forces to make the pump mechanism operable.

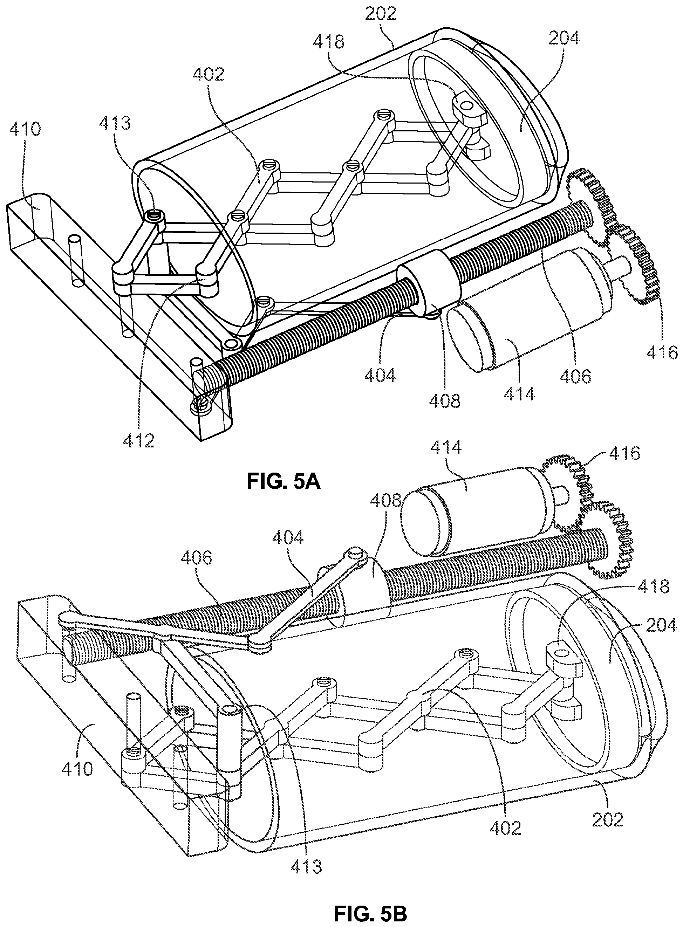

**FIGS. 5A-5B** show a variation of the embodiment of **FIGS. 4A-4D** in which the connection point 412 between linkage 404 and scissor mechanism 402 is disposed on the opposite side of scissor mechanism 402 from connection point 412 of the embodiment of **FIGS. 4A-4D****.** Note that, in the embodiment of **FIGS. 4A-4D****,** the linkage 404 extends to the side of reservoir 202, thereby allowing the overall mechanism to have a smaller height profile at the expense of having a larger width profile. The variation of the embodiment shown in **FIGS. 5A-5B** has the opposite effect, wherein the linkage 404 extends underneath (or above) reservoir 202, thereby increasing the height profile but decreasing the width profile of the mechanism. A cross-sectional shape of the reservoir 202 can be adapted so as to accommodate linkage 404 as it moves below (or above) reservoir 202. For example, a bottom (or top) of reservoir 202 can be planar to accommodate motion of linkage 404, and the plunger 204 can be similarly modified in cross-sectional shape to correspond to the modified cross-sectional shape of the reservoir 202 (e.g., modified from a circular or oval cross-section).

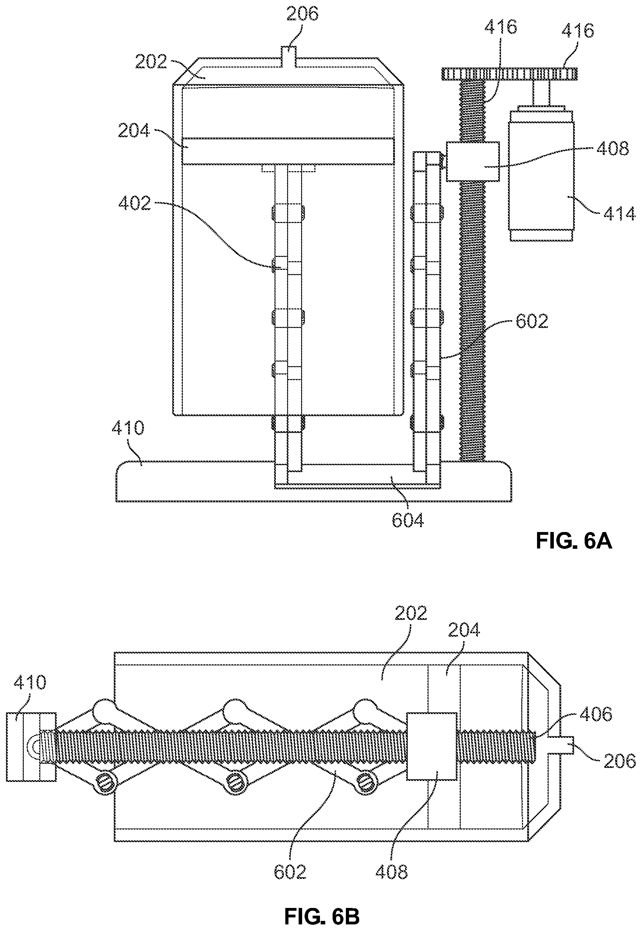

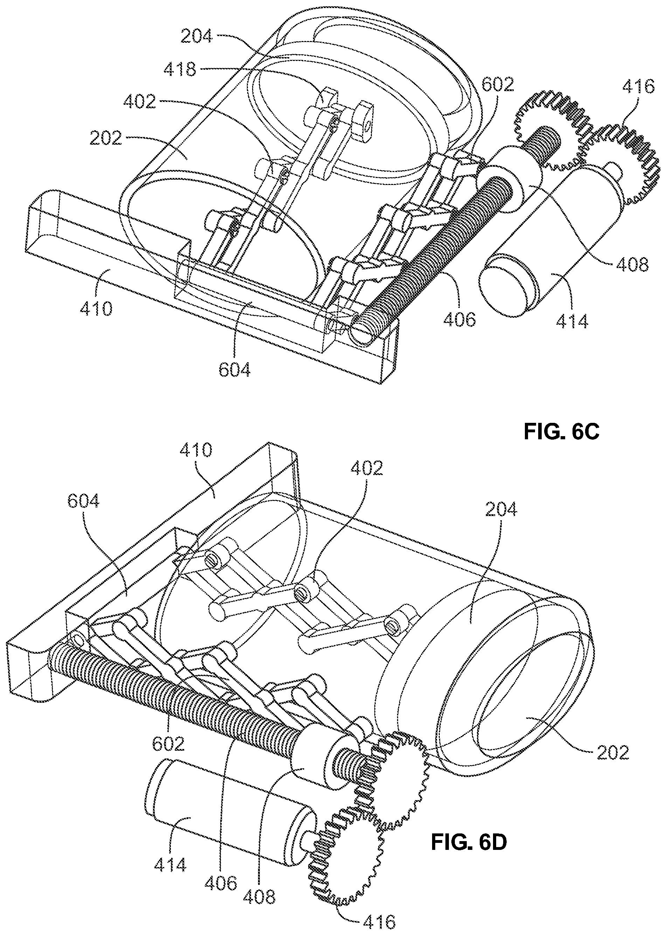

**FIGS. 6A-6D** show several views of a second embodiment of the invention in which linkage 404 is replaced by a second scissor mechanism 602 which is coupled, at one end, directly to drive nut 408. At the opposite end, second scissor mechanism 602 is coupled to scissor mechanism 402 via linkage 604, best shown in **FIG. 6D****.** In this embodiment, reservoir 202 and linkage 604 should both be rigidly attached to the body 410 of the device, such that reservoir 202 and linkage 604 are unable to move with respect to each other. As such, movement of drive nut 408 in the direction of the body 410 of the device will cause scissor mechanism 402 to contract while movement of drive nut 408 in a direction away from the body 410 of the device will cause scissor mechanism 402 to expand, thereby driving plunger 204 toward the distal end of reservoir 202. All other aspects of the second embodiment shown in **FIGS. 6A-6D** are identical to those shown in the preferred embodiment in **FIGS. 4A-4D****.**

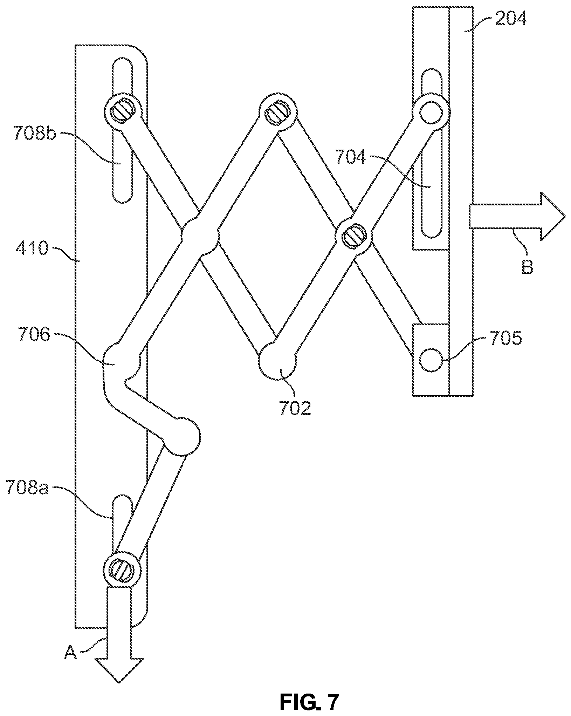

**FIG. 7** shows an alternate linkage in which one upper segment of scissor mechanism 702 is coupled to plunger 204 via a sliding mechanism 704 and the other upper segment of the scissor mechanism 702 is rotationally coupled to plunger 204 at fixed point 705. The lower segments of scissor mechanism 702 are coupled to the body 410 of the device via sliding mechanisms 708a, 708b such that movement of scissor mechanism 702 in the direction shown by arrow "A" causes plunger 204 to move in the direction shown by arrow "B". One advantage to this arrangement is that the drive mechanism may be oriented so as to provide the required force in a direction parallel to the direction shown by arrow "A", as opposed to other embodiments, wherein the drive mechanism imparts a force in a direction parallel with the direction of arrow "B". For example, in the case where the drive mechanism comprises a leadscrew and a drive nut, the leadscrew (not shown) may be oriented perpendicular to the longitudinal axis of reservoir 202.

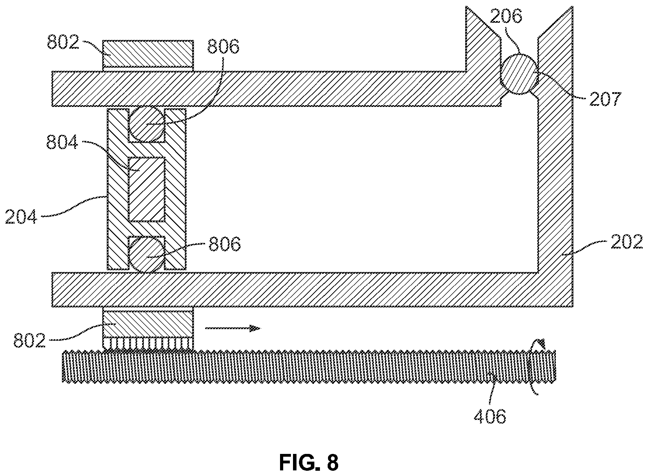

**FIG. 8** shows a third embodiment of the invention in which plunger 204 is configured with a first magnet 804. Preferably, first magnet 804 is molded into, or otherwise securely coupled to an underside of, plunger 204. The pump mechanism further comprises a second magnet 802 which is disposed along an outside surface of reservoir 202, such that movement of the second magnet 802 in a longitudinal direction along the outside surface of reservoir 202 will cause first magnet 804 to move in the same longitudinal direction based on a magnetic engagement between second magnet 802 and first magnet 804. The movement of the first magnet 804 in the longitudinal direction toward the distal end of reservoir 202 will drive plunger 204 in the longitudinal direction toward the distal end of reservoir 202. O-Ring 806 may be provided to seal the interface between plunger 204 and the inside wall of reservoir 202.

In this embodiment, the second magnet 802 may be configured as a collar surrounding the outside surface of reservoir 202. Second magnet 802 may be guided as it moves longitudinally along the outside surface of reservoir 202 by one or more guides (not shown) which may be, for example, ridges or channels defined on the outside surface of reservoir 202.

In this embodiment, the drive mechanism may comprise any mechanism configured to cause second magnet 804 to move longitudinally along the outer surface of reservoir 202. In preferred embodiments, the drive mechanism comprises a leadscrew 406 and wherein second magnet 802 has outside threads which are in threaded engagement with leadscrew 406. Second magnet 802 conforms to and encircles the outside surface of reservoir 202 such that second magnet 802 is able to slide in the longitudinal direction along the outside surface of reservoir 202 when driven by the rotation of leadscrew 406.

As with other embodiments herein, leadscrew 406 may be rotationally driven via any known means, including, for example, a motor (not shown) coupled directly or via gearing to leadscrew 406. The advantage of the embodiment shown in **FIG. 8** over the other embodiments discussed which use a scissor mechanism, is that overall length of the pump mechanism in this embodiment may be shorter because the space required to store the scissor mechanism when it is in a contracted position may not be necessary.

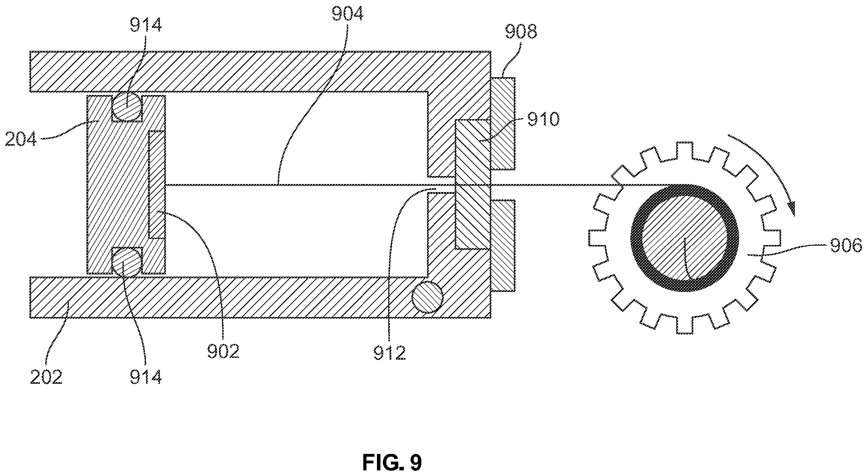

**FIG. 9** shows a fourth embodiment of the invention. In this embodiment, plunger 204 is coupled to tether 904 which extends through a sealed opening 912 in the distal end of reservoir 202. In this embodiment, the drive mechanism comprises a take-up reel 906 which rotates in a manner such as to wind tether 904 about take-up reel 906, thereby drawing plunger 204 toward the distal end of reservoir 202. Tether 904 may pass through one or more seals 908, 910 which prevent the liquid drug from leaking out of reservoir 202 through opening 912 and which may be composed of, for example, silicone or rubber. In some embodiments, tether 904 may be a metal or polymer wire or film connected to take-up reel 906. One advantage of this embodiment is that take-up reel 906 may be disposed at any location within the body or housing of the drug delivery device and may use one or more pulleys to direct tether 904 through opening 912, thereby allowing flexibility with respect to the use of space within the device 100. O-Ring 914 may be provided to seal the interface between plunger 204 and the inside wall of reservoir 202.

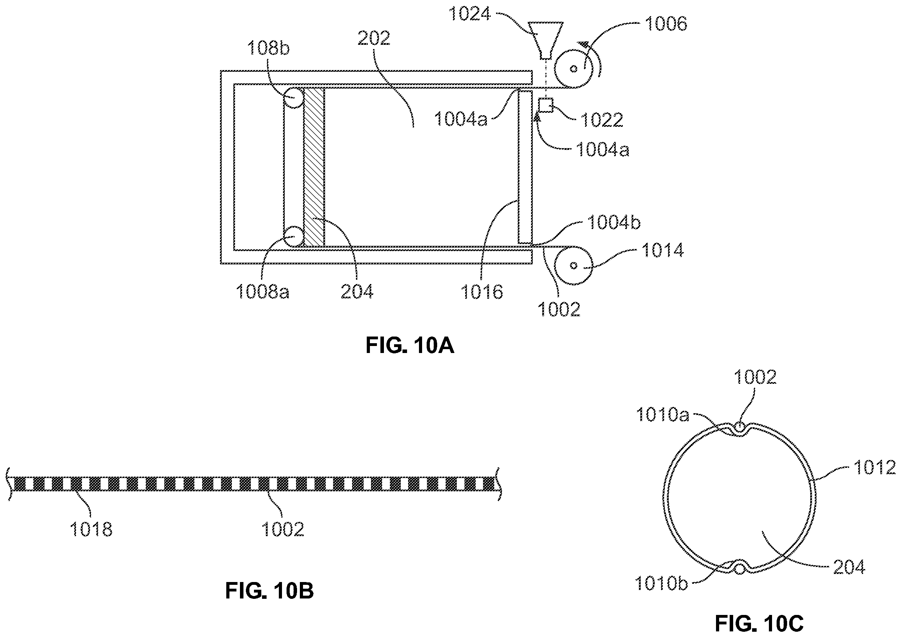

**FIGS. 10****(A-C)** show a fifth embodiment of the invention similar to the fourth embodiment shown in **FIG. 9****.** In this embodiment, shown in a cross-sectional view in **FIG. 10A****,** plunger 204 is coupled to tether 1002 which extends through two sealed openings 1004(a,b) in the distal end of reservoir 202. Similar to the fourth embodiment, the drive mechanism comprises a take-up reel 1006 which rotates in a manner such as to wind tether 1002 about take-up reel 1006, thereby "pushing" plunger 204 from behind toward the distal end of reservoir 202. Tether 1002 may pass through two or more seals (not shown) in openings 1004(a,b) , which prevent the liquid drug from leaking out of reservoir 202 through openings 1004(a,b) and which may be composed of, for example, silicone or rubber. In some embodiments, tether 1002, shown in more detail in **FIG. 10B****,** may be a metal or polymer wire, film or tape connected to take-up reel 1006. Tether 1002 may extend through cutouts 1010(a,b) in plunger 204 and may be sealed by O-ring 1012 which extends around the outer edge of plunger 204. Additional seals (not shown) may be required in cutouts 1010(a,b) to prevent the liquid drug from leaking through cutouts 1010(a,b). Tether 1002 may be anchored at the end opposite the end connected to take-up reel 1006 at anchor point 1014. In a variation of this embodiment, opening 1004b may be eliminated by anchoring tether 1002 to the interior surface of distal wall 1016 of reservoir 202.

In this embodiment, plunger 204 is pulled by exerting a force on the back side of plunger 204, for example, on opposite ends of plunger 204, at stationary pulleys 1008(a,b), as shown in **FIG. 10A****.** Similar to the fourth embodiment described above, an advantage of this embodiment is that take-up reel 1006 may be disposed at any location within the body or housing of the drug delivery device and may use one or more pulleys to direct tether 1002 through opening 1004a, thereby allowing flexibility with respect to the use of space within the device 100.

Tether 1002 may be provided with a means to alternately pass and block light therethrough. In one embodiment, tether 1002 mat be provided with holes 1018 at regularly spaced intervals, as shown in **FIG. 10B****,** wherein the holes 1018 allow light to pass through tether 1002 and the areas between the holes 1018 prevent light from passing through tether 1002. In other embodiments, tether 1002 may be a film have alternating light and dark areas, wherein the light areas allow light to pass through tether 1002 and the dark areas block light from passing through tether 1002. Note that the tether 1002 described with respect to the embodiment of **FIGS. 10A-C** may also be used with the embodiment of **FIG. 9****.**

The embodiment of **FIGS. 10A-C,** as well as the embodiment shown in **FIG. 9****,** may further include a light source 1022, for example, an LED, and a light sensor 1024 or scanner component connected to circuitry and a processor that is programmed to count a number of light flashes. As tether 1002 alternately passes and blocks light from passing therethrough as it is pulled toward take-up reel 1006, the number of light flashes can be counted by sensing the light flashes with sensor 1024, and, thereby, the processor may determine how much of tether 1002 has passed by the sensor 1024. In this manner, because the spacing between the holes 1018 in (or the light and dark areas of) tether 1002 is known, it may be determined how far plunger 204 has traveled, and accordingly, how much liquid drug has been dispensed.

Further, when reservoir 202 is being filled with a liquid drug by a user, light source 1022 and sensor 1024 may be operative to determine how much liquid drug has been inserted into reservoir 204. As the user injects the liquid drug through an inlet (not shown), tether 1002 may unwind from take-up reel 1006. As the holes 1018 in the tether 1002 pass (to the left in **FIG. 10A**) between light source 1022 and sensor 1024, sensor 1024 may count the number of light flashes it senses as light from the light source 1022 passes through the regularly spaced holes 1018 in tether 1002. Based on the count of the number of light flashes, the processor may determine how much tether 1002 has been unwound from take-up reel 1006, and in this manner, may determine how far plunger 204 has traveled while reservoir 202 is being filled, and accordingly, how much liquid drug the user has inserted into reservoir 202. As such, tether 1002 with regularly spaced holes 1018 may function as a "fuel gauge" to determine not only how much liquid drug has been dispensed, but also how much liquid drug has been inserted into the reservoir 202 and, by extension, how much liquid drug remains in reservoir 202. Such information may be communicated wirelessly (e.g., via Bluetooth) to a remote device having a user interface, such that the user may know each of these variables: how much liquid drug has been inserted into reservoir 202; how much liquid drug has been dispensed at any point in time; and how much liquid drug remains in reservoir 202.

The following examples pertain to various embodiments of the pump mechanism suitable for use in a wearable drug delivery device:
Example 1 is a first embodiment of a pump mechanism comprising a reservoir, a plunger disposed in the reservoir and a scissor mechanism coupled to the plunger for moving the plunger longitudinally through the interior of the reservoir.
Example 2 is an extension of Example 1, or any other example disclosed herein, wherein the pump mechanism further comprises a drive mechanism coupled to the scissor mechanism for expanding and contracting the scissor mechanism.
Example 3 is an extension of Example 2, or any other example disclosed herein, wherein the drive mechanism comprises a leadscrew and a drive nut in threaded engagement with the leadscrew, the drive nut being coupled to the scissor mechanism.
Example 4 is an extension of Example 3, or any other example disclosed herein, wherein the drive nut is coupled to the scissor mechanism via a linkage.
Example 5 is an extension of Example 4, or any other example disclosed herein, when the scissor mechanism is connected at one end to the plunger and at the other end to the body of the device.
Example 6 is an extension of Example 5, or any other example disclosed herein, wherein the reservoir and one end of the scissor mechanism are coupled to the body such as to prevent relative movement therebetween.
Example 7 is a second embodiment of a pump mechanism comprising a reservoir, a plunger disposed in the reservoir, a first scissor mechanism coupled to the plunger for moving the plunger longitudinally through the interior of the reservoir and a second scissor mechanism coupled to the first scissor mechanism via a linkage.
Example 8 is an extension of Example 7, or any other example disclosed herein, wherein the pump mechanism further comprises a drive mechanism coupled to the second scissor mechanism.
Example 9 is an extension of Example 8, or any other example disclosed herein, wherein the drive mechanism comprises a leadscrew in threaded engagement with a drive nut and wherein the drive nut is coupled to the second scissor mechanism.
Example 10 is an extension of Example 9, or any other example disclosed herein, wherein the reservoir and the linkage are coupled to the body of the device such as to prevent relative movement therebetween.
Example 11 is a third embodiment of a pump mechanism comprising a reservoir, a plunger disposed in the reservoir and coupled to a first magnet, a second magnet disposed along the outside surface of the reservoir, and a drive mechanism coupled to the second magnet for driving the second magnet along the outside surface of the reservoir.
Example 12 is an extension of Example 11, or any other example disclosed herein, wherein the first and second magnets are in magnetic engagement with each other such that movement of the second magnet along the outside surface of the reservoir causes longitudinal movement of first magnet and, thereby, the plunger, in the interior of the reservoir.
Example 13 is an extension of Example 12, or any other example disclosed herein, wherein the second magnet is in the shape of a collar disposed around the outside circumference of the reservoir.
Example 14 is an extension of Example 13, or any other example disclosed herein, wherein outside surface of the reservoir is configured with ridges to guide movement of the second magnet along the outside surface of the reservoir.
Example 15 is extension of Example 11, or any other example disclosed herein, wherein the outside surface of the second magnet is configured with threads and further wherein the second magnet is in threaded engagement with the leadscrew such that rotational motion of the leadscrew is translated to linear motion of the second magnet.
Example 16 is an extension of Example 11, or any other example disclosed herein, wherein the reservoir and drive mechanism are rigidly attached to the body of a device such as to prevent relative movement therebetween.
Example 17 is a fourth embodiment of a pump mechanism comprising a reservoir, a plunger disposed in the reservoir, a tether coupled to the plunger and extending through a sealed opening in the closed end of the reservoir, and a drive mechanism for pulling the tether through the sealed opening.
Example 18 is an extension of Example 17, or any other example disclosed herein, wherein the drive mechanism comprises a take-up reel coupled to the tether such that rotation of the take-up reel will cause a tether to be pulled through the sealed opening of the reservoir.
Example 19 is an extension of Example 18, or any other example disclosed herein, wherein the pump mechanism further comprises one or more pulleys to guide the tether from the take-up reel to the sealed opening in the reservoir.
Example 20 is an extension of Example 17, or any other example disclosed herein, wherein the reservoir and the drive mechanism are rigidly connected to the body of the device such as to prevent relative movement therebetween.
Example 20 is an extension of Example 17, or any other example disclosed herein, wherein the tether is coupled to an interior surface of the plunger.
Example 21 is an extension of Example 17, or any other example disclosed herein, wherein the tether is anchored at one end and coupled to an exterior surface of the plunger via one or more pulleys.

To those skilled in the art to which the invention relates, many modifications and adaptations of the invention may be realized. Implementations provided herein, including sizes, shapes, ratings and specifications of various components or arrangements of components, and descriptions of specific manufacturing processes, should be considered exemplary only and are not meant to limit the invention in any way. As one of skill in the art would realize, many variations on implementations discussed herein which fall within the scope of the invention are possible. Moreover, it is to be understood that the features of the various embodiments described herein were not mutually exclusive and can exist in various combinations and permutations, even if such combinations or permutations were not made express herein, without departing from the spirit and scope of the invention. Accordingly, the method and apparatus disclosed herein are not to be taken as limitations on the invention but as an illustration thereof. The scope of the invention is defined by the claims which follow.
Although the present invention is defined in the attached claims, it should be understood that the present invention can also (alternatively) be defined in accordance with the following embodiments:
1. A pump mechanism comprising:
   a reservoir comprising a tube-like structure having one open end and one closed end, the closed end configured with a fluid path;
   a plunger, disposed in the reservoir, the plunger configured move longitudinally within the reservoir; and
   a scissor mechanism coupled to the plunger and extending out through the open end of the reservoir.
2. The pump mechanism of embodiment 1, further comprising:
   a drive mechanism coupled to the scissor mechanism for driving the scissor mechanism between a contracted position and an expanded position;
   wherein movement of the scissor mechanism from the contracted position to the expanded position causes the plunger to move longitudinally in the reservoir toward the closed end of the reservoir.
3. The pump mechanism of embodiment 2 wherein the drive mechanism comprises:
   a leadscrew; and
   a drive nut in threaded engagement with the leadscrew such that rotational motion of the leadscrew translates to a linear motion of the drive nut;
   wherein the drive nut is coupled to the scissor mechanism.
4. The pump mechanism of embodiment 3 further comprising:
   a linkage coupling the drive nut to the scissor mechanism.
5. The pump mechanism of embodiment 1 wherein:
   the scissor mechanism is rotatably coupled to the plunger at one end; and
   the scissor mechanism is rotatably coupled to a body of a device at an opposite end.
6. The pump mechanism of embodiment 5 wherein the reservoir is coupled to the body of the device such that the reservoir and the end of the scissor mechanism coupled to the body of the device are unable to move with respect to each other.
7. A pump mechanism comprising:
   a reservoir comprising a tube-like structure having one open end and one closed end, the closed end configured with a fluid path;
   a plunger, disposed in the reservoir, the plunger configured move longitudinally within the reservoir;
   a first scissor mechanism coupled to the plunger and extending out through the open end of the reservoir; and
   a second scissor mechanism coupled to the first scissor mechanism via a linkage.
8. The pump mechanism of embodiment 7, further comprising:
   a drive mechanism coupled to the second scissor mechanism for driving the scissor mechanism between a contracted position and an expanded position;
   wherein movement of the second scissor mechanism from the contracted position to the expanded position movement of the first scissor mechanism from the contracted position to the expanded position, thereby causing the plunger to move longitudinally in the reservoir toward the closed end of the reservoir.
9. The pump mechanism of embodiment 8 wherein the drive mechanism comprises:
   a leadscrew; and
   a drive nut in threaded engagement with the leadscrew such that rotational motion of the leadscrew translates to a linear motion of the drive nut;
   wherein the drive nut is coupled to the second scissor mechanism.
10. The pump mechanism of embodiment 9 wherein the reservoir and the linkage are coupled to a body of a device such that the reservoir and the linkage are unable to move with respect to each other.
11. A pump mechanism comprising:
   a reservoir comprising a tube-like structure having one open end and one closed end, the closed end configured with a fluid path;
   a plunger, disposed in the reservoir, the plunger configured move longitudinally within the reservoir;
   a first magnet, coupled to the plunger;
   a second magnet, external to the reservoir and able to move longitudinally along an outside surface of the reservoir; and
   a drive mechanism coupled to the second magnet for driving the second magnet along the outside surface of the reservoir.
12. The pump mechanism of embodiment 11, wherein movement of the second magnet longitudinally along the outside surface of the reservoir causes the first magnet, and thus the plunger, to move longitudinally through the interior of the reservoir based on a magnetic engagement between the second magnet and the first magnet.
13. The pump mechanism of embodiment 12 wherein the second magnet is in the shape of a collar surrounding the outside surface of the reservoir;
14. The pump mechanism of embodiment 13 wherein the outside surface of the reservoir is configured with one or more guide ridges to keep the second magnet in alignment as it moves along the outside surface of the reservoir.
15. The pump mechanism of embodiment 11 wherein the drive mechanism comprises:
   a leadscrew;
   wherein:
      an outside surface of the second magnet is configured with threads;
      the second magnet and leadscrew are in threaded engagement with each other;
      rotation of the leadscrew in a first direction will cause movement of the second magnet in a first longitudinal direction along the outside surface of the reservoir; and
      rotation of the leadscrew in a second direction will cause movement of the second magnet in a second longitudinal direction along the outside surface of the reservoir.
16. The pump mechanism of embodiment 11 wherein the reservoir and the drive mechanism are rigidly attached to a body of a device such that the reservoir and drive mechanism are unable to move with respect to each other.
17. A pump mechanism comprising:
   a reservoir comprising a tube-like structure having one open end and one closed end, the closed end configured with a fluid path;
   a plunger, disposed in the reservoir, the plunger configured move longitudinally within the reservoir;
   a tether, extending through a sealed opening in the closed end of the reservoir and coupled to the plunger; and
   a drive mechanism, for pulling the tether through the sealed opening, thereby causing the plunger to move toward the closed end of the reservoir.
18. The pump mechanism of embodiment 17 wherein the drive mechanism comprises a take-up reel, coupled to the tether, such that rotation of the take-up reel will cause the tether to be pulled through the sealed opening of the reservoir.
19. The pump mechanism of embodiment 18 further comprising:
   one or more pulleys to guide the tether from the take-up reel to the sealed opening.
20. The pump mechanism of embodiment 17 wherein the reservoir and the drive mechanism are rigidly connected to a body of the device such that the reservoir and the drive mechanism are unable to move with respect to each other.
21. The pump mechanism of embodiment 17 wherein the tether is coupled to a surface of the plunger interior to the reservoir.
22. The pump mechanism of embodiment 17 wherein an end of the tether opposite an end coupled to the drive mechanism is anchored and further wherein the tether is coupled to the plunger via one or more pulleys mounted on a surface of the plunger exterior to the reservoir.

## Claims

1. A pump mechanism comprising:
a reservoir comprising a tube-like structure having one open end and one closed end, the closed end configured with a fluid path;
a plunger, disposed in the reservoir, the plunger configured move longitudinally within the reservoir;
a tether, extending through a sealed opening in the closed end of the reservoir and coupled to the plunger; and
a drive mechanism, for pulling the tether through the sealed opening, thereby causing the plunger to move toward the closed end of the reservoir.

2. The pump mechanism of claim 1 wherein the drive mechanism comprises a take-up reel, coupled to the tether, such that rotation of the take-up reel will cause the tether to be pulled through the sealed opening of the reservoir, in particular wherein the drive mechanism comprises one or more pulleys to guide the tether from the take-up reel to the sealed opening and/or through the opening.

3. The pump mechanism of claim 2 wherein the take-up reel is disposed at a location within a housing of the device.

4. The pump mechanism of claims 1 wherein the reservoir and the drive mechanism are rigidly connected to a body of the device such that the reservoir and the drive mechanism are unable to move with respect to each other.

5. The pump mechanism of claim 1 wherein the tether is coupled to a surface of the plunger interior to the reservoir.

6. The pump mechanism of claim 1 wherein an end of the tether opposite an end coupled to the drive mechanism is anchored and further wherein the tether is coupled to the plunger via one or more pulleys mounted on a surface of the plunger exterior to the reservoir.

7. The pump mechanism of claim 1 wherein the tether passes through one or more seals configured to prevent leakage of liquid from the reservoir.

8. The pump mechanism of claim 1 wherein the tether extends through cutouts in the plunger.

9. The pump mechanism of claim 1, further comprising a light source and a light sensor or scanner component connected to circuitry and a processor, wherein the tether is provided with a means to alternately pass and block light therethrough, and wherein the light sensor or scanner component is operative to detect light from the light source as the tether moves, such that a number of light flashes is counted and it is determined how much liquid drug has been inserted into and/or dispensed from the reservoir.

10. The pump mechanism of claim 9 provided with holes at regularly spaced intervals, wherein the holes allow light to pass through tether and the areas between the holes prevent light from passing through tether and/or wherein the tether is a film having alternating light and dark areas, the light areas allowing light to pass through the tether and the dark areas blocking light from passing through the tether.

11. A pump mechanism comprising:
a reservoir comprising a tube-like structure having one open end and one closed end, the closed end configured with a fluid path;
a plunger, disposed in the reservoir, the plunger configured move longitudinally within the reservoir;
a first magnet, coupled to the plunger;
a second magnet, external to the reservoir and able to move longitudinally along an outside surface of the reservoir; and
a drive mechanism coupled to the second magnet for driving the second magnet along the outside surface of the reservoir.

12. The pump mechanism of claim 11, wherein movement of the second magnet longitudinally along the outside surface of the reservoir causes the first magnet, and thus the plunger, to move longitudinally through the interior of the reservoir based on a magnetic engagement between the second magnet and the first magnet, in particular wherein the second magnet is in the shape of a collar surrounding the outside surface of the reservoir.

13. The pump mechanism of claim 12 wherein the outside surface of the reservoir is configured with one or more guide ridges to keep the second magnet in alignment as it moves along the outside surface of the reservoir.

14. The pump mechanism of claim 11 wherein the drive mechanism comprises:
a leadscrew;
wherein:
an outside surface of the second magnet is configured with threads;
the second magnet and leadscrew are in threaded engagement with each other;
rotation of the leadscrew in a first direction will cause movement of the second magnet in a first longitudinal direction along the outside surface of the reservoir; and
rotation of the leadscrew in a second direction will cause movement of the second magnet in a second longitudinal direction along the outside surface of the reservoir.

15. The pump mechanism of claim 11 wherein the reservoir and the drive mechanism are rigidly attached to a body of a device such that the reservoir and drive mechanism are unable to move with respect to each other.
